# EUROPEAN PATENT SPECIFICATION

(11) **EP 1 532 953 B1**
(45) Date of publication and mention of the grant of the patent: **04.08.2010**
(21) Application number: 03733491.9
(22) Date of filing: 18.06.2003
(51) Int. Cl.: A61F 13/15

(54) **METHOD OF PRODUCING DISPOSABLE DIAPER**
VERFAHREN ZUR HERSTELLUNG EINER EINWEG-WINDEL
METHODE DE PRODUCTION D'UNE COUCHE JETABLE

(30) Priority: 04.07.2002 JP 2002196080
(43) Date of publication of application: 25.05.2005
(73) Proprietor: UNI-CHARM CO., LTD., Kawanoe, Ehime 799-0111 (JP)
(72) Inventor: GODA, Hidefumi, c/o UNI-CHARM CO., LTD, Mitoyo-gun, Kagawa 769-1602 (JP); TAKEUCHI, Kenji, c/o UNI-CHARM CO., LTD, Mitoyo-gun, Kagawa 769-1602 (JP)
(74) Representative: Sperling, Rüdiger
(86) International application number: PCT/JP2003/007737
(87) International publication number: WO 2004/004618

(56) References cited:
- EP-A- 0 291 598
- EP-A1- 0 048 011
- EP-A2- 0 396 050
- JP-A- 4 161 152
- JP-A- 4 161 152
- JP-A- 6 296 638
- JP-A- 63 296 865
- US-A- 4 646 510
- US-A- 4 795 510
- US-A- 5 756 039

## Description

### TECHNICAL FIELD OF THE INVENTION

The present invention relates to a process for making disposable diapers.

### BACKGROUND ART OF THE INVENTION

Japanese Patent Application Publication No. 1992-161152A discloses a process for making absorbent articles such as disposable diapers. According to the process disclosed in this Publication, a web sheet which is a series of individual absorbent bodies and extends in a machine direction is coated on one surface with an adhesive agent and then this web sheet is guided between a roll serving as a cutting means and a roll serving as a reorienting means and cut to obtain the individual absorbent bodies. The individual absorbent bodies are successively rotated around their own axes on the reorienting means by 90° from the machine direction to a transverse direction orthogonal to the machine direction and then joined to the outermost sheet fed in the machine direction so that the individual absorbent bodies are placed on the outermost sheet intermittently in the machine direction.

In the above-cited known process, the roll serving as a cutting means is provided on its peripheral surface with a blade extending in an axial direction of the roll and commonly called as an upper tool. The roll serving as a reorienting means has its peripheral surface which the upper tool hits and is commonly called as a lower tool. The peripheral surface of the roll serving as a cutting means has a relatively small circumferential dimension which usually corresponds to a length of one or two individual absorbent bodies. The roll serving as a reorienting means has a circumferential length sufficient to support at least two individual absorbent bodies on its peripheral surface and to allow one of these two individual absorbent bodies to rotate around its own axis. With a consequence, in many cases, the roll serving as a reorienting means has a diameter as large as three to five times of the diameter of the roll serving as a cutting means. Since the blade and the peripheral surface which the blade hits are liable to be worn during continuous use of these two rolls, these two rolls need periodic maintenance and checks as well as periodic exchanges of parts. Regarding this, the roll serving also as the lower cutter requires much time and labor for maintenance, checks and parts-exchanges because of its relatively large diameter. The hot melt type adhesive agent commonly used in the process for making a disposable diaper may also raise a problem. Depending on a viscosity at which the hot melt type adhesive agent is applied, a layer of the coated adhesive agent may be unnecessarily thick. In consequence, the article such as diaper may be uncomfortably stiff.

It is an object of the present invention to provide a process for continuously making a disposable diaper improved so as to facilitate maintenance, checks and parts-changes of upper and lower tools to make a thickness of an adhesive layer light.

### DISCLOSURE OF THE INVENTION

According to the present invention, there is provided a process for continuously making disposable diapers (1) comprising the steps of
- coating the outer surface of a continuous backsheet (322) of a first web (351) with an adhesive agent, said first web (351) being fed in a first machine direction (A) and including said backsheet (322), a continuous topsheet (321) and a plurality of blocks (323) made of body fluid absorbent material and interposed between said two sheets (322, 321) and arranged intermittently in said first machine direction (A),
- cutting said first web (351) in a transverse direction (C) orthogonal to said first machine direction (A) between respective pairs of adjacent said blocks (323) to obtain individual absorbent members (302),
- reorienting said individual absorbent members (302) from said first machine direction (A) to said transverse direction (C) and
- bonding said individual absorbent members (302) to a second web (352) comprising a continuous inner sheet (304) fed in a second machine direction (B) orthogonal to said transverse direction (C) so that at least a part of each of said individual absorbent members (302) constitutes a crotch region (8) of said diaper (1) and at least a part of said second web (352) constitutes a waist region (6, 7) of said diaper.

The improvement according to the present invention is **characterized in that**
- an adhesive agent is a hot melt adhesive agent (354); and
- said first web (351) coated with a hot melt adhesive agent (354) is guided between upper and lower tool rolls (361, 362) extending in said transverse direction (C) in parallel to each other,
- said first web (351) is cut by these rolls(361, 362) between respective pairs of adjacent said blocks (323) to obtain said individual absorbent members (302),
- said absorbent members (302) are then guided between said lower tool roll (362) and a guide roll(363) extending in said transverse direction (C) in parallel to each other with a hot melt adhesive agent (354) on said backsheet (322) pressed against a peripheral surface of said guide roll (363),
- then said individual absorbent members (302) are transferred from said guide roll(363) to a reorienting roll(364) extending in parallel to said guide roll(363) with said topsheet (321) placed against a peripheral surface of said reorienting roll (364),
- said individual absorbent members (302) are successively reoriented from said first machine direction (A) to said transverse direction (C) on said reorienting roll (364) and
- then said individual absorbent members (302) are fed onto said second web (352) and bonded to said second web (352) by means of said hot melt adhesive agent (354).

Advantageous features of the process are in the dependent claims.

### BRIEF DESCRIPTION OF THE DRAWINGS

Fig. 1 is a partially cutaway plan view showing a disposable diaper;
Fig. 2 is a partially cutaway perspective view showing the disposable diaper of an arrangement different from the arrangement shown in Fig. 1; and
Fig. 3 is a diagram illustrating a part of the process for making the disposable diaper.

### DESCRIPTION OF THE BEST MODE FOR WORKING OF THE INVENTION

Details of the process according to the present invention for making disposable diaper will be more fully understood from the description given hereunder with reference to the accompanying drawings.

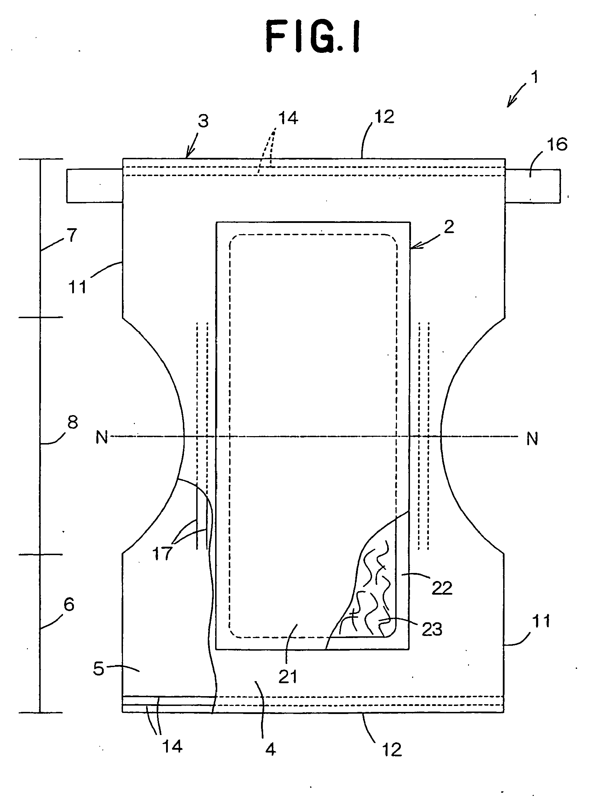

Fig. 1 is a partially cutaway plan view showing an open-type disposable diaper 1 according to this invention. The diaper 1 generally comprises a body fluid absorbent member 2 having a rectangular shape dimensioned to be longer in a longitudinal direction of the diaper 1 and an hourglass-shaped sheet-like support member 3. Configurationally, the diaper 1 is composed of, as viewed in the longitudinal direction, a front waist region 6, a rear waist region 7 and a crotch region 8 extending between these two waist regions 6, 7. A dimension of the diaper 1 in the longitudinal direction is bisected by a center line N-N.

The support member 3 includes an inner sheet 4 facing a wearer's body and an outer sheet 5 facing a wearer's undergarment, both being adhesively or thermally joined to each other. The support member 3 at least partially defines the front waist region 6, the rear waist region 7 and the crotch region 8. In the front and rear waist regions 6, 7, waist-surrounding elastic members 14 extend in a transverse direction of the diaper 1 between these inner and outer sheets 4, 5 and are secured in a stretched state to at least one of these sheets 4, 5. The inner and outer sheets 4, 5 are liquid-pervious or liquid-impervious and made of a suitable material such as a plastic film or a nonwoven fabric.

The body fluid absorbent member 2 includes a liquid-pervious topsheet 21, liquid-impervious or liquid-pervious backsheet 22 and a block 23 which is interposed between these top- and backsheets 21, 22 and made of body absorbent material. The topsheet 21 and the backsheet 22 are overlaid and joined together outside along a peripheral edge of the block 23 using a suitable adhesive agent or a heat-sealing technique. The body fluid absorbent member 2 in which the block 23 is covered with the topsheet 21 and the backsheet 22 in this manner extends in the crotch region 8 further into the front and rear waist regions 6, 7 wherein the backsheet 22 is bonded to the inner sheet 4 by means of an adhesive agent (not shown). In this manner, the body fluid absorbent member 2 makes a part of these respective regions 6, 7, 8. In the case of the backsheet 22 which is liquid-pervious, the inner sheet 4 or the outer sheet 5 is preferably liquid-impervious. The disposable diaper constructed as has been described above can be worn in same manner as a well-known open-type disposable diaper.

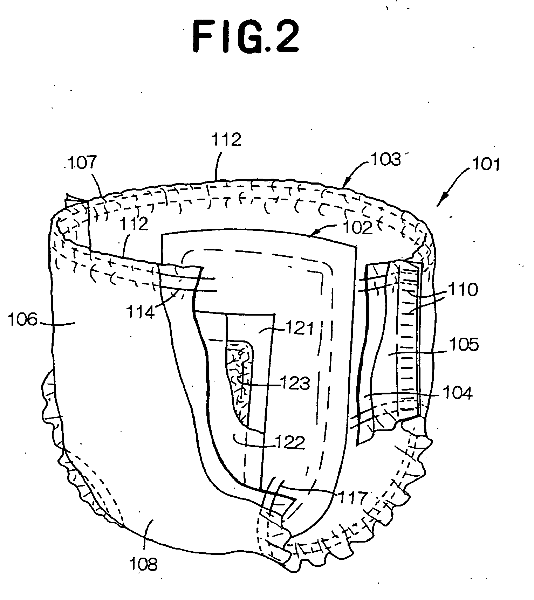

Fig. 2 is a partially cutaway perspective view showing a pants-type disposable diaper 101 obtained by the process according to the invention. The diaper 101 comprises a pants-shaped support member 103 and a body fluid absorbent member 102 attached to an inner side of the support member 103. The support member 103 at least partially defines a front waist region 106, a rear waist region 107 and a crotch region 108. The front and rear waist regions 106, 107 are overlaid and joined together at a plurality of joining lines 110. Such the diaper 101 can be obtained by folding the diaper 1 of Fig. 1 with the body fluid absorbent member 2 inside along the center line N-N and then joining the front and rear waist regions 6, 7 together in the vicinity of transversely opposite side edge zones 11 of the respective waist regions 6, 7. However, tape fasteners 16 as seen in Fig. 1 are unnecessary to obtain the diaper 101 of Fig. 2 from the diaper 1 of Fig. 1 and may be previously eliminated. The regions, portions and zones in the diaper 101 of Fig. 2 which correspond to those in the diaper 1 of Fig. 1 are designated by the reference numerals in Fig. 1 added with 100, respectively.

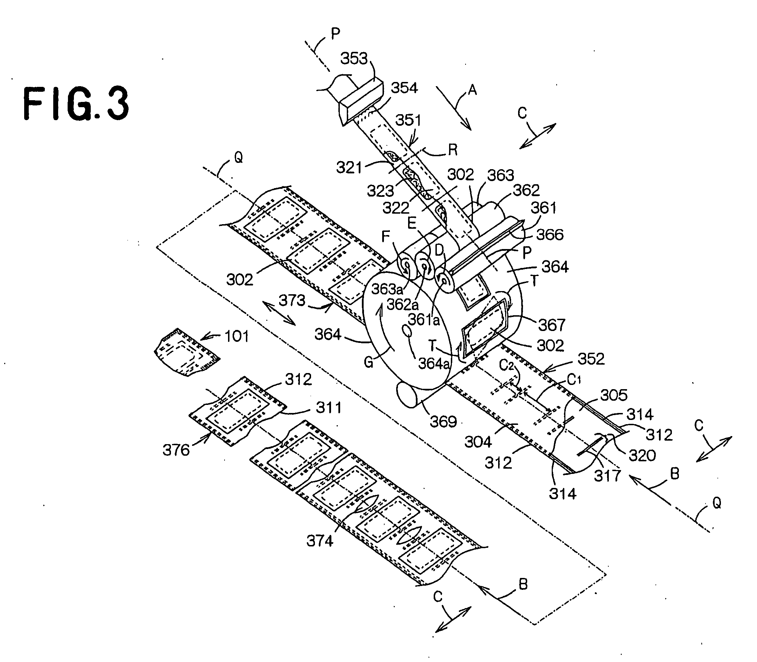

Fig. 3 is a perspective diagram illustrating a part of the process for continuously making the diaper 1 or the diaper 101. In the upper part of the diagram, a first web 351 is fed in a first machine direction indicated by an arrow A and, in the lower part of the diagram, a second web 352 is fed in a second machine direction indicated by an arrow B. A direction indicated by an arrow C which is orthogonal to the first machine direction A is orthogonal also to the second machine direction B. While center lines P, Q extending to bisect the first web 351 and the second web 352, respectively, in the transverse direction C are illustrated to lie on the same plane orthogonal to the transverse direction C, it is also possible to arrange these two center lines P, Q so as to be spaced one from another in the transverse direction C. Of these first and second webs 351, 352, the second web 352 comprises a continuous inner sheet 304 and a continuous outer sheet 305 placed upon and joined to each other. Between the continuous inner sheet 304 and the continuous outer sheet 305, a plurality of first elastic members 314 continuously extending in a stretched state in the second machine direction B and a plurality of second elastic members 317 extending in a stretched state in a transverse direction of the second web 352, i.e., in the transverse direction C are interposed. The first elastic members 314 lie in the vicinity of transversely opposite side edges 312 extending in parallel to each other and are bonded to at least one of the sheets 304, 305. A plurality of second elastic members 317 lie in a group 320 in a transversely middle zone of the second web 352 so that

these groups 320 are spaced one from another in the second machine direction B alternately by a relatively small dimension C₂ and a relatively large dimension C₁. The second elastic members 317 are bonded to at least one of the sheets 304, 305.

In the upper part of Fig. 3, the first web 351 comprising a continuous topsheet 321, a continuous backsheet 322 and an absorbent block 323 interposed between these two sheets 321, 322 is illustrated to be fed in the first machine direction A. As illustrated, the absorbent blocks 323 each having a rectangular shape which is relatively long in the first machine direction A and arranged intermittently in the first machine direction A. The continuous topsheet 321 and the continuously backsheet 322 are overlaid and joined together outside along peripheral edges of the respective blocks 323 by use of a suitable adhesive agent or a heat-sealing technique. Such the first web 351 is fed with its continuous backsheet 322 facing upward in the first machine direction A and, in the course of being fed in this manner, the continuous backsheet 322 is coated by an adhesive coater 353 with a hot melt adhesive agent 354 in a desired pattern.

The first web 351 fed in the first machine direction A and the second web 352 fed in the second machine direction B are integrated together by means of an upper tool roll 361, Q lower tool roll 362, a guide roll 363, a reorienting roll 364 and a press roll 369 as shown in Fig. 3. The upper tool roll 361 and the lower tool roll 362 respectively rotate in directions indicated by arrows D, E around axes 361a, 362a extending in parallel to each other in a direction orthogonal to the first machine direction A. The upper tool roll 361 is provided on its peripheral surface with a blade 366 extending in parallel to the axis 361a so that the blade 366 may come in contact with a smooth peripheral surface of the lower tool roll 362 under an adjustable pressure. The lower tool roll 362 is formed on its peripheral surface with a plurality of vacuum suction openings (not shown). The lower tool roll 362 and the guide roll 363 respectively rotate in directions indicated by arrows E, F around axes 362a, 363a extending in parallel to each other so that one of these axes 362a, 363a may be shifted to adjust a distance between these two rolls 362, 363 and/or to adjust a contact pressure of the rolls 362, 363 when these two rolls 362, 363 rotate with the first web 351 interposed therebetween. A peripheral surface of the guide roll 363 is formed by material such as a silicone rubber or a fluorocarbon resin so that the hot melt adhesive agent 354 in a molten or semi-molten state may be easily separated from the peripheral surface. In addition, this peripheral surface is temperature-adjustable so that the hot melt adhesive agent 354 may be maintained in adhesively effective condition and separated from the peripheral surface in a stable condition. The guide roll 363 is also provided on its peripheral surface with a plurality of vacuum suction openings (not shown). The reorienting roll 364 extends in parallel to the guide roll 363 and rotates around on an axis 364a in a direction indicated by an arrow G. The reorienting roll 364 is provided on its peripheral surfaces with a plurality of rotary tables 367 intermittently arranged around the axis 364a. While details are not illustrated, each of these rotary tables 367 rotates on the peripheral surface of the roll 364 around this table's own axis by 90° in a direction indicated by an arrow T during 1/2 rotation of the reorienting roll 364 in the direction G and then rotates on the peripheral surface of the roll 364 around this table's own axis by 90° in a direction opposite to the direction T during the subsequent 1/2 rotation of the roll 364. The axis 363a of the guide roll 363 may be shifted to ensure that the guide roll 363 is brought in contact with the peripheral surfaces of the rotary tables 367 or spaced apart from the peripheral surface of the rotary tables 367 by a desired dimension.

The first web 351 is fed in the first machine direction toward these rolls 361, 362, 363 and 364. The first web 351 having the continuous backsheet 322 coated with the hot melt adhesive agent 354 and facing upward is first guided between the upper tool roll 361 and the lower tool roll 362. The first web 351 fed in close contact with the peripheral surface of the lower tool roll 362 under the vacuum suction is then transferred onto the guide roll 363. On the peripheral surface of the lower tool roll 362, the first web 351 is cut by the blade 366 of the upper tool roll 361 along an imaginary line R transversely extending between each pair of the adjacent blocks 323, 323 made of an absorbent material into the individual body fluid absorbent members 302. The individual body fluid absorbent members 302 are pressed by the lower tool roll 362 against the peripheral surface of the guide roll 363 and at the same time retained on this peripheral surface of the guide roll 363 under the vacuum suction from this roll 363. Then the individual body fluid absorbent members 302 are guided to the reorienting roll 364. The hot melt adhesive agent 354 in a molten or semi-molten condition is pressed against the peripheral surface of the guide roll 363 as the body fluid absorbent members 302 are pressed against the peripheral surface of the guide roll 363. Consequently, the hot melt adhesive agent 354 is flattened out on the entire surface of the backsheet 322 and a layer of the adhesive agent 354 is correspondingly thinned. The vacuum suction from the lower tool roll 362 is stopped when the body fluid absorbent member 302 is separated from the lower tool roll 362.

The vacuum suction from the guide roll 363 is stopped when the body fluid absorbent member 302 on the guide roll 363 comes in contact with the associated rotary table 367 on the reorienting roll 364 and at the same time this body fluid absorbent member 302 is transferred with the backsheet 322 facing upward to the associated rotary table 367 under the vacuum suction from the vacuum suction openings (not shown) of the rotary table 367.

The reorienting roll 364 is opposed to the guide roll 363 lying immediately above the reorienting roll 364 and opposed to the press roll 369 lying immediately below the reorienting roll 364 as viewed in the vertical direction in Fig. 3. During 1/2 rotation of the reorienting roll 364 around the axis 364a from the guide roll 363 toward the press roll 369 in the direction G, the rotary table 367 rotates around its own axis by 90° in the direction T. So the body fluid absorbent member 302 oriented heretofore in the first machine direction A, i.e., in the direction orthogonal to the axis 364a of the reorienting roll 364 is reoriented so as to extend in parallel to the axis 364a, i.e., in the transverse direction C. The body fluid absorbent member 302 is squeezed together with the second web 352 between these two rolls 364, 369 as the reorienting roll 364 is opposed to the press roll 369 to join the body fluid absorbent member 302 to the continuous inner sheet 304 of the second web 352 by means of the hot melt adhesive agent 354 and thereby to form a third web 373. In this third web 373, each of the body fluid absorbent members 302 is interposed between each pair of the adjacent groups 320, 320 of the second elastic members 317 spaced apart from each other by the relatively large dimension C₁. The vacuum suction from the rotary table 367 is stopped when the associated body fluid absorbent member 302 is joined to the second web 352.

The third web 373 is further fed in the second machine direction B and subjected to a step of cutting out. Specifically, substantially disc-shaped portions of the inner and outer sheets 304, 305 placed upon each other are successively cut out from the third web 373 to form openings 374 between each pair of the adjacent groups 320, 320 of the second elastic members 317 spaced apart from each other by the relatively small dimension C₂. The third web 373 is then cut between each pair of adjacent groups 320, 320 of the second elastic members 317 spaced apart from each other by the relatively small dimension C₂ thereby to form individual assemblies 376. Each of the assemblies 376 is contoured by longitudinally opposite ends 312 corresponding to the transversely opposite side edges 312 continuously extending in the second machine direction B and transversely opposite side edges 311 extending in the direction C orthogonal to the second machine direction B. The side edges 311 are defined by the bisected disc-shaped zones so as to describe circular arcs curving inward, respectively.

The assembly 376 may be provided in the vicinity of one of the longitudinally opposite ends 312 with tape fasteners (not shown) extending outward from the transversely opposite side edges 311, respectively. The assembly 376 obtained in this manner corresponds to the diaper 1 of Fig. 1 in which the inner sheet 4 and the outer sheet 5 are defined by the inner sheet 304 and the outer sheet 305 in the assembly 376, respectively, and the absorbent member 2 is defined by the body fluid absorbent member 302. The tape fasteners 16 in Fig. 1 are defined by the tape fasteners with which the assembly 376 may be provided.

The assembly 376 may be folded with the body fluid absorbent member 302 inside in the transverse direction C, then the inner and outer sheets 304, 305 may be overlaid and joined together in the vicinity of the transversely opposite side edges 311, respectively, to obtain the pants-type diaper 101 of Fig. 2. In this case, the inner and outer sheets 304, 305 are destined to define the inner and outer sheets 104, 105 in Fig. 2. The body fluid absorbent member 302 is destined to define the absorbent member 102 in Fig. 2.

With the process according to the invention for continuously making the disposable diaper as has been described above, a circumferential dimension of the upper tool roll 361 may be set to be substantially equal to the body fluid absorbent member 302 as measured in the first machine direction A to minimize a diameter of the upper tool roll 361. While the lower tool roll 362 and the guide roll 363 may have respective diameters smaller than the diameter of the upper tool roll 361, it is preferred that these rolls 362, 363 have the diameters substantially equal to the diameter of the upper tool roll 361. In view of the fact that the reorienting roll 364 must have a plurality of rotary tables 367 and these rotary tables 367 must rotate their own axes, this reorienting roll 364 usually has a diameter as large as three to five times of the diameter of the upper tool roll 361. It should be understood here that the present invention allows the diameters of the upper tool roll 361 and the lower tool roll 362 to be reduced independently of the diameter of the reorienting roll 364 in order to facilitate maintenance and/or check of these rolls, exchange of the blade 366, mounting and/or demounting of these rolls. Furthermore, the invention allows the layer of the hot melt adhesive agent 354 to be pressed against the easily-releasable peripheral surface of the guide roll 363 and thereby to be flattened out over the entire surface of the backsheet 322. Such the layer of the adhesive agent 354 is not only thin and flexibly deformable but also allows the body fluid absorbent member 302 to be bonded to the second web 352 over an area as large as possible. The body fluid absorbent member 302 and the second web 352 may be squeezed between the reorienting roll 364 and the press roll 369 to press the layer of the hot melt adhesive agent 354 applied on the body fluid absorbent member 302 to be pressed against the second web 352. In this case, however, it is apprehended that the hot melt adhesive agent 354 might adhere to the continuous backsheet 322 of the body fluid absorbent member 302 made of nonwoven fabric or plastic film and the continuous inner sheet 304 of the second web 352. This phenomenon makes it difficult to flatten out and thereby to thin the layer of the hot melt adhesive agent 354.

The invention is not limited to the illustrated embodiment and without departing from the scope of the invention it is possible to attach the elastic members in a stretched state so as to extend in the first machine direction A to the side edges of the first web 351. The second web 352 may be replaced by the web formed by nonwoven fabric or plastic film having none of the first elastic members 314 and the second elastic members 317 bonded thereto. While the first machine direction A and the second machine direction B are illustrated in the accompanying diagram to be opposite to each other, it is also possible to coincide these two machine directions with each other.

The process according to the present invention for making the disposable diaper is primarily characterized in that the first web is cut between the upper and lower tool rolls to obtain the individual body fluid absorbent members which are then squeezed between the lower tool roll and the guide roll and then fed onto the reorienting roll. Such a unique process allows the diameters of the upper and lower tool rolls to be reduced in order to facilitate maintenance and check of these rolls as well as exchange of the parts. The layer of the hot melt adhesive agent applied on the individual body fluid absorbent member is pressed against the peripheral surface of the guide roll and thereby flatten out and thinned. The disposable diaper obtained according to this process is free from the inconvenience that the hot melt adhesive agent layer might result in a feeling of stiffness and become undesirably thick.

## Claims

1. A process for continuously making disposable diapers (1) comprising the steps of
• coating the outer surface of a continuous backsheet (322) of a first web (351) with an adhesive agent, said first web (351) being fed in a first machine direction (A) and including said backsheet (322), a continuous topsheet (321) and a plurality of blocks (323) made of body fluid absorbent material and interposed between said two sheets (322, 321) and arranged intermittently in said first machine direction (A),
• cutting said first web (351) in a transverse direction (C) orthogonal to said first machine direction (A) between respective pairs of adjacent said blocks (323) to obtain individual absorbent members (302),
• reorienting said individual absorbent members (302) from said first machine direction (A) to said transverse direction (C) and
• bonding said individual absorbent members (302) to a second web (352) comprising a continuous inner sheet (304) fed in a second machine direction (B) orthogonal to said transverse direction (C) so that at least a part of each of said individual absorbent members (302) constitutes a crotch region (8) of said diaper (1) and at least a part of said second web (352) constitutes a waist region (6, 7) of said diaper,
said process being **characterized in that:**
• an adhesive agent is a hot melt adhesive agent (354); and
• said first web (351) coated with a hot melt adhesive agent (354) is guided between upper and lower tool rolls (361, 362) extending in said transverse direction (C) in parallel to each other,
• said first web (351) is cut by these rolls(361, 362) between respective pairs of adjacent said blocks (323) to obtain said individual absorbent members (302),
• said absorbent members (302) are then guided between said lower tool roll (362) and a guide roll(363) extending in said transverse direction (C) in parallel to each other with a hot melt adhesive agent (354) on said backsheet (322) pressed against a peripheral surface of said guide roll (363),
• then said individual absorbent members (302) are transferred from said guide roll(363) to a reorienting roll(364) extending in parallel to said guide roll(363) with said topsheet (321) placed against a peripheral surface of said reorienting roll (364),
• said individual absorbent members (302) are successively reoriented from said first machine direction (A) to said transverse direction (C) on said reorienting roll (364) and
• then said individual absorbent members (302) are fed onto said second web (352) and bonded to said second web (352) by means of said hot melt adhesive agent (354).

2. The process according to Claim 1, wherein said peripheral surface of said guide roll (363) is adapted to be temperature-adjustable.

3. The process according to Claim 1 or 2, wherein diameters of said upper and lower tool rolls (3361, 362) are equal to each other and smaller than that of said reorienting roll(364).

4. The process according to any one of Claims 1 through 3, wherein said first web (351) fed in said first machine direction (A) and said second web (352) fed in said second machine direction (B) are integrated together by means of said upper tool roll (361), said lower tool roll (362), said guide roll (363), a reorienting roll (364) and a press roll (369). [source: column 5, line 25-29]

5. The process according to any one of Claims 1 through 4, wherein said upper tool roll (361) is provided on its peripheral surface with a blade (366) extending in parallel to the axis (361 a) so that said blade (366) comes in contact with a smooth peripheral surface of the lower tool roll (362) under an adjustable pressure. [source: column 5, line 34-38]

6. The process according to any one of Claims 1 through 5, wherein said lower tool roll (362) is formed on its peripheral surface with a plurality of vacuum suction openings and wherein said guide roll (363) is provided on its peripheral surface with a plurality of vacuum suction openings. [source: column 5, line 38-40 and column 5, line 56-58]

7. The process according to any one of Claims 1 through 6, wherein said lower tool roll (362) and said guide roll (363) respectively rotate around axes (362a, 363a) extending in parallel to each other so that one of these axes (362a, 363a) is shiftable to adjust a distance between these two rolls (362, 363) and/or to adjust a contact pressure of the rolls (362, 363). [source: column 5, line 40-46]

8. The process according to any one of Claims 1 through 7, wherein rotary tables (367) rotate on the peripheral surface of the roll (364) around this table's own axis by 90° in a T-direction during ½ rotation of the reorienting roll (364) in a G-direction. [source: column 6, line 6-10]

9. The process according to any one of Claims 1 through 8, wherein said individual body fluid absorbent members (302) are pressed by the lower tool roll (362) against the peripheral surface of the guide roll (363) and at the same time retained on this peripheral surface of the guide roll (363) under the vacuum suction from this roll (363). [source: column 6, line 32-37]

10. The process according to any one of Claims 1 through 9, wherein said hot melt adhesive agent (354) in a molten or semi-molten condition is pressed against the peripheral surface of the guide roll (363) as the body fluid absorbent members (302) are pressed against the peripheral surface of the guide roll (363). [source: column 6, line 39-43]

11. The process according to any one of Claims 1 through 10, wherein said body fluid absorbent member (302) is squeezed together with the second web (352) between the reorienting roll (364) and the press roll (369) to join the body fluid absorbent member (302) to the continuous inner sheet (304) of the second web (352) by means of the hot melt adhesive agent (354) and thereby to form a third web (373). [source: column 7, line 13-20]

12. The process according to Claim 11, wherein said third web (373) is fed in the second machine direction (B) and subjected to a step of cutting out, and wherein disc-shaped portions of the inner and outer sheets (304, 305) placed upon each other are successively cut out from the third web (373) to form openings (374) between each pair of the adjacent groups (320, 320) of the second elastic members (317) spaced apart from each other by a relatively small dimension C₂. [source: column 7, line 28-36]

13. The process according to Claim 91 or 12, wherein said third web (373) is cut between each pair of adjacent groups (320, 320) of the second elastic members (317) spaced apart from each other by the relatively small dimension C₂ thereby to form individual assemblies (376). [source: column 7, line 36-40]

14. The process according to any one of Claims 1 through 13, wherein said lower tool roll (362) and said guide roll (363) have respective diameters smaller than the diameter of the upper tool roll (361). [source: column 8, line 19-21]

15. The process according to any one of Claims 1 through 14, wherein said reorienting roll (364) has a plurality of rotary tables (367) and a diameter as large as three to five times of the diameter of the upper tool roll (361). [source: column 8, line 24-25 and column 8, line 26-28]

## Patentansprüche

1. Verfahren zur kontinuierlichen Herstellung von Wegwerfwindeln (1), enthaltend die Schritte
• Beschichten der Außenfläche einer Endlos-Außenlage (322) einer ersten Bahn (351) mit einem Klebstoff, welche erste Bahn (351) in einer ersten Maschinenrichtung (A) zugeliefert wird und die Außenlage (322), eine Endlos-Decklage (321) und eine Vielzahl von Blöcken (323) umfasst, die aus Körperflüssigkeit absorbierendem Material hergestellt sind und zwischen die beiden Lagen (322, 321) gelegt sind und in der ersten Maschinenrichtung (A) in Abständen angeordnet sind,
• Schneiden der ersten Bahn (321) in Querrichtung (C) senkrecht zu der ersten Maschinenrichtung (A) zwischen jeweiligen Paaren von benachbarten Blöcken (323), um einzelne absorbierende Elemente (302) zu erhalten,
• Neuausrichtung der einzelnen absorbierenden Elemente (302) aus der ersten Maschinenrichtung (A) in die Querrichtung (C) und
• Verbinden der einzelnen absorbierenden Elemente (302) mit einer zweiten Bahn (352), die eine Endlos-Innenlage (304) aufweist, die in einer zweiten Maschinenrichtung (B) senkrecht zu der Querrichtung (C) zugeliefert wird, so dass zumindest ein Tell jedes der einzelnen absorbierenden Elemente (302) einen Schrittbereich (8) der Windel (1) bildet und zumindest ein Teil der zweiten Bahn (352) einen Hüftbereich (6, 7) der Windel bildet,
welches Verfahren **dadurch gekennzeichnet ist, dass**:
• ein Klebstoff ein Helßschmelzklebstoff (354) ist; und
• die mit einem Heißschmelzklebstoff (354) beschichtete erste Bahn (351) zwischen einer oberen und einer unteren Werkzeugwalze (361, 362) geführt wird, die parallel zueinander in der Querrichtung (C) verlaufen,
• die erste Bahn (351) von diesen Walzen (361, 362) zwischen jeweiligen Paaren von benachbarten Blöcken (323) geschnitten wird, um die einzelnen absorbierenden Elemente (302) zu erhalten,
• die absorbierenden Elemente (302) dann zwischen der unteren Werkzeugwalze (362) und einer Führungswalze (363) geführt werden, die in der Querrichtung (C) parallel zueinander verlaufen, wobei ein Heißschmelzklebstoff (354) auf der Außenlage (322) gegen eine Umfangsfläche der Führungswalze (363) gepresst wird,
• anschließend die einzelnen absorbierenden Elemente (302) von der Führungswalze (363) zu einer Neuausrichtungswalze (364) übertragen werden, welche parallel zu der Führungswalze (363) verläuft, wobei die Decklage (321) auf einer Umfangsfläche der Neuausrichtungswalze (364) platziert wird,
• die einzelnen absorbierenden Elemente (302) auf der Neuausrichtungswalze (364) aufeinander folgend aus der ersten Maschinenrichtung (A) in die Querrichtung (C) neu ausgerichtet werden und
• anschließend die einzelnen absorbierenden Elemente (302) auf die zweite Bahn (352) zugeliefert werden und mit der zweiten Bahn (352) mittels des Heißschmelzklebstoffes (354) verbunden werden.

2. Verfahren nach Anspruch 1, bei welchem die Umfangsfläche der Führungswalze (363) dafür ausgelegt ist, dass ihre Temperatur regelbar ist.

3. Verfahren nach Anspruch 1 oder 2, bei welchem Durchmesser der oberen und der unteren Werkzeugwalze (361, 362) einander gleich sind und kleiner als der der Neuausrichtungswalze (364),

4. Verfahren nach einem der Ansprüche 1-3, bei welchem die erste Bahn (351), die in der ersten Maschinenrichtung (A) zugeliefert wird, und die zweite Bahn (352), die in der zweiten Maschinenrichtung (B) zugeliefert wird, mittels der oberen Werkzeugwalze (361), der unteren Werkzeugwalze (362), der Führungswalze (363), einer Neuausrichtungswalze (364) und einer Presswalze (369) miteinander verbunden werden. [Quelle: Spalte 5, Zelle 25-29]

5. Vorfahren nach einem der Ansprüche 1-4, bei welchem die obere Werkzeugwalze (361) an ihrer Umfangsfläche mit einem parallel zu der Achse (361a) verlaufenden Messer (366) versehen ist, so dass das Messer (366) mit einer glatten Umfangfläche der unteren Werkzeugwalze (362) unter einem einstellbaren Druck in Berührung kommt. [Quelle: Spalte 5, Zelle 34-38]

6. Verfahren nach einem der Ansprüche 1-5, bei welchem die untere Werkzeugwalze (362) mit einer Vielzahl von Vakuum-Saugöffnungen auf ihrer Umfangsfläche gebildet ist und bei welchem die Führungswalze (363) auf ihrer Umfangsfläche mit einer Vielzahl von Vakuum-Saugöffnungen versehen ist. [Quelle: Spalte 5, Zeile 38-40 und Spalte 5, Zelle 56-58]

7. Verfahren nach einem der Ansprüche 1-6, bei welchem die untere Werkzeugwalze (362) und die Führungswalze (363) jeweils um parallel zueinander verlaufende Achsen (362a, 363a) umlaufen, wobei eine dieser Achsen (362a, 363a) verschlebbar ist, um einen Abstand zwischen diesen beiden Walzen (362, 363) einzustellen und/oder einen Berührungsdruck der Walzen (362, 363) einzustellen. [Quelle: Spalte 5, Zeile 40-46]

8. Verfahren nach einem der Ansprüche 1-7, bei welchem während einer halben Umdrehung der Neuausrichtungswalze (364) in einer G-Richtung Drehtische (367) auf der Umfangsfläche der Walze (364) um 90° in einer T-Richtung um die eigene Achsen dieser Tische drehen. [Quelle: Spalte 6, Zeile 6-10]

9. Verfahren nach einem der Ansprüche 1-8, bei welchem die einzelnen Körperflüssigkeit absorbierenden Elemente (302) durch die untere Werkzeugwalze (362) gegen die Umfangsfläche der Führungswalze (363) gepresst werden und gleichzeitig unter Vakuumsaugwirkung von dieser Walze (363) an dieser Umfangsfläche der Führungswalze (363) gehalten werden. [Quelle: Spalte 6, Zeile 32-37]

10. Verfahren nach einem der Ansprüche 1-9, bei welchem der Heißschmelzklebstoff (354) in einem geschmolzenen oder halb geschmolzenen Zustand gegen die Umfangsfläche der Führungswalze (363) gepresst wird, wenn das Körperflüssigkeit absorbierende Element (302) gegen die Umfangsfläche der Führungswalze (363) gepresst wird. [Quelle: Spalte 6, Zeile 39-43]

11. Verfahren nach einem der Ansprüche 1-10, bei welchem das Körperflüssigkeit absorbierende Element (302) zwischen der Neuausrichtungswalze (364) und der Anpresswalze (369) mit der zweiten Bahn (352) zusammengedrückt wird, um das Körperflüssigkeit absorbierende Element (302) mit der Endlos-Innenlage (304) der zweiten Bahn (352) mittels des Heißschmelzklebstoffes (354) zu verbinden und **dadurch** eine dritte Bahn (373) zu bilden. [Quelle: Spalte 7, Zeile 13-20]

12. Verfahren nach Anspruch 11, bei welchem die dritte Bahn (373) in der zweiten Maschinenrichtung (B) zugeliefert wird und einem Ausschneideschritt unterzogen wird, und bei welchem scheibenförmige Abschnitte der inneren und der äußeren Lage (304, 305), die übereinander angeordnet sind, aufeinander folgend aus der dritten Bahn (373) ausgeschnitten werden, um Öffnungen (374) zwischen jedem Paar von benachbarten Gruppen (320, 320 der zweiten elastischen Elemente (317) zu bilden, die voneinander mit einem relativ geringen Maß C₂ beabstandet sind. [Quelle: Spalte 7, Zeile 28-36]

13. Verfahren nach Anspruch 11 oder 12, bei welchem die dritte Bahn (373) zwischen jedem Paar von benachbarten Gruppen (320, 320) der zweiten elastischen Elemente (317) geschnitten wird, die voneinander mit dem relativ geringen Maß C₂ beabstandet sind, um **dadurch** einzelne Anordnungen (376) zu bilden. [Quelle: Spalte 7, Zeile 36-40]

14. Verfahren nach einem der Ansprüche 1-13, bei welchem die untere Werkzeugwalze (362) und die Führungswalze (363) jeweils Durchmesser haben, die kleiner als der Durchmesser der oberen Werkzeugwalze (361) sind. [Quelle: Spalte 8, Zeile 19-21]

15. Verfahren nach einem der Ansprüche 1-13, bei welchem die Neuausrichtungswalze (364) eine Vielzahl von Drehtischen (367) und einen Durchmesser hat, der das Drel- bis Fünffache des Durchmessers der oberen Werkzeugwalze (361) ist. [Quelle: Spalte 8, Zeile 24-25 und Spalte 8, Zeile 26 -28]

## Revendications

1. Procédé pour la réalisation en continu de couches jetables (1) comprenant les étapes suivantes :
- revêtement de la surface externe d'une feuille externe continue (322) d'une première bande (351) avec un agent adhésif, ladite première bande (351) étant alimentée dans une première direction de machine (A) et incluant ladite feuille externe (322), une feuille de dessus continue (321) et une pluralité de blocs (323) faits d'un matériau absorbant le liquide corporel et interposés entre lesdits deux feuilles (322, 321) et disposés par intermittence dans ladite première direction de machine (A),
- coupe de ladite première bande (351) dans une direction transversale (C) orthogonale à la dite première direction de machine (A) entre les paires respectives de dits blocs adjacents (323) pour obtenir des organes absorbants individuels (302),
- réorientation desdits organes absorbants individuels (302) depuis ladite première direction de machine (A) vers ladite direction transversale (C), et
- la liaison desdits organes absorbants individuels (302) à une seconde bande (352) comprenant une première feuille interne continue (304) alimentée dans une seconde direction de machine (B) orthogonale à ladite direction transversale (C) de sorte qu'au moins une partie de chacun des dits organes individuels absorbants (302) constitue une région d'entrejambe (8) de ladite couche (1) et au moins une partie de ladite seconde bande (352) constitue une région de taille (6, 7) de ladite couche,
ledit procédé étant **caractérisé en ce**
- **qu**'un agent adhésif est un agent adhésif thermofusible (354); et
- ladite première bande (351) revêtue d'un agent adhésif thermofusible (354) est guidée entre des rouleaux d'outil supérieurs et inférieurs (361, 362) s'étendant dans la direction transversale (C) parallèlement entre eux,
- ladite première bande (351) est coupée par ces rouleaux (361, 362) entre lesdites paires respectives desdits blocs adjacents (323) pour obtenir lesdits organes absorbants individuels (302),
- lesdits organes (302) sont alors guidés entre ledit rouleau d'outil inférieur (362) et un rouleau de guidage (363) s'étendant dans ladite direction transversale (C) parallèlement entre eux avec un agent adhésif thermofusible (354) sur ladite feuille arrière (322) pressée contre une surface périphérique dudit rouleau de guidage (363),
- lesdits organes individuels absorbants (302) sont transférés dudit rouleau de guidage (363) à un rouleau de réorientation (364) s'étendant parallèlement au dit rouleau de guidage (363) avec ladite feuille de dessus (321) placée contre une surface périphérique dudit rouleau de réorientation (364),
- lesdits organes absorbants individuels (302) sont réorientés successivement depuis ladite première direction de machine (A) vers ladite direction transversale (C) sur ledit rouleau de réorientation (364) et
- ensuite lesdits organes absorbants individuels (302) sont alimentés sur ladite seconde bande (352) et sont liés à ladite seconde bande (352) au moyen dudit agent adhésif thermufusible (354).

2. Procédé selon la revendication 1, dans lequel ladite surface périphérique dudit rouleau de guidage (363) est ajustable en température.

3. Procédé selon la revendication 1 ou 2, dans lequel des diamètres desdits rouleaux supérieurs et inférieurs d'outil (361, 362) sont égaux entre eux et plus petits que celui dudit rouleau de réorientation (364).

4. Procédé selon l'une des revendications 1 à 3, dans lequel ladite première bande (351) alimentée dans ladite première direction de machine (A) et ladite seconde bande (352) alimentée dans ladite seconde direction de machine (B) sont intégrées ensemble au moyen dudit rouleau supérieur d'outil (361), dudit rouleau inférieur d'outil (362), dudit rouleau de guidage d'outil (363), d'un rouleau de réorientation (364) et d'un rouleau de pression (369) [Source: colonne 5, ligne 25-29].

5. Procédé selon l'une des revendications 1 à 4, dans lequel ledit rouleau d'outil supérieur (361) est muni sur sa surface périphérique d'une lame (366) s'étendant parallèlement à l'axe (361a) de sorte que ladite lame (366) vient en contact avec une surface périphérique glissante dudit rouleau Inférieur d'outil (362) sous une pression réglable [Source: colonne 5, ligne 34-38].

6. Procédé selon l'une des revendications 1 à 5, dans lequel ledit rouleau Inférieur d'outil (362) possède sur sa surface périphérique une pluralité d'ouvertures d'aspiration de vide et dans lequel ledit rouleau de guidage (363) est muni sur sa surface périphérique d'une pluralité d'ouvertures d'aspiration de vide [Source: colonne 5, ligne 38-40 et colonne 5, ligne 56-58].

7. Procédé selon l'une des revendications de 1 à 6, dans lequel ledit rouleau inférieur d'outil (362) et ledit rouleau de guidage (363) tournent respectivement autour d'axes (362a, 363a) s'étendant parallèlement entre eux de sorte que l'un de ces axes (362a, 363a) est ripable pour ajuster une distance entre ces deux rouleaux (362, 363) et/ou pour ajuster une pression de contact des rouleaux (362, 363). [Source: colonne 5, ligne 40-46].

8. Procédé selon l'une des revendications de 1 à 7, dans lequel des tables rotatives (367) tournent sur la surface périphérique de la bobine (364) autour de ce propre axe de la table selon un angle de 90° dans une direction T pendant une demi-rotation du rouleau de réorientation (364) dans une direction G [Source: colonne 6, ligne 6-10].

9. Procédé selon l'une des revendications de 1 à 8, dans lequel lesdits organes absorbants individuels de liquide corporel (302) sont pressés par le rouleau d'outil inférieur (362) contre la surface périphérique du rouleau de guidage (363) et en même temps sont retenus sur cette surface périphérique du rouleau de guidage (363) sous une aspiration de vide depuis ce rouleau (363). [Source: colonne 6, ligne 32-37].

10. Procédé selon l'une des revendications de 1 à 9, dans lequel ledit agent adhésif thermofusible (354) dans un état fondu ou semi-fondu est pressé contre la surface périphérique du rouleau de guidage (363) lorsque les organes absorbants de liquide corporel (302) sont pressés contre la surface périphérique du rouleau de guidage (363) [Source: colonne 6, ligne 39-43].

11. Procédé selon l'une des revendications de 1 à 10, dans lequel ledit organe absorbant de liquide corporel (302) est serré ensemble avec la seconde bande (352) entre le rouleau de réorientation (364) et le rouleau de pression (369) pour relier l'organe absorbant de liquide corporel (302) à la feuille interne continue (304) de la seconde bande (352) au moyen de l'agent adhésif thermofusible (354) et pour former ainsi une troisième bande (373). [Source: colonne 7, ligne 13-20].

12. Procédé selon la revendication 11, dans lequel ladite troisième bande (373) est alimentée dans la seconde direction de machine (B) et soumise à une étape de coupe et dans lequel des portions en forme de disque des feuilles internes et externes (304, 305) placées les unes sur les autres sont coupées successivement de la troisième bande (373) pour former des ouvertures (374) entre chaque paire des groupes adjacents (320, 320) des seconds organes élastiques (317) séparés les uns des autres par une dimension relativement petite C₂ [Source: colonne 7, ligne 28-36].

13. Procédé selon la revendication 11 ou 12, dans lequel ladite troisième bande (373) est coupée entre chaque paire de groupes adjacents (320, 320) des secondes organes élastiques (317) séparés par la dimension relativement petite C₂ pour former des structures individuelles (376) [Source: colonne 7, ligne 36-40].

14. Procédé selon l'une des revendications 1 à 13, dans lequel ledit rouleau inférieur d'outil (362) et ledit rouleau de guidage (363) ont des diamètres respectifs plus petits que le diamètre dudit rouleau d'outil supérieur (361) [Source: colonne 8, ligne 19-21].

15. Procédé selon l'une des revendications de 1 à 14, dans lequel ledit rouleau de réorientation (364) a une pluralité de tables rotatives (367) et un diamètre trois à cinq fois plus large que le diamètre du rouleau supérieur d'outil (361) [Source: colonne 8, ligne 24-25 et colonne 8, ligne 26-28].
